# EUROPÄISCHE PATENTSCHRIFT

(11) **EP 1 089 719 B1**
(45) Veröffentlichungstag und Bekanntmachung des Hinweises auf die Patenterteilung: **10.04.2002**
(21) Anmeldenummer: 99927904.5
(22) Anmeldetag: 08.06.1999
(51) Int. Cl.: A61K 9/70, A61K 31/57, A61K 31/565, A61K 47/18, A61L 15/22, A61L 15/44, A61L 15/58

(54) **TRANSDERMALES THERAPEUTISCHES SYSTEM, ENTHALTEND HORMONE UND KRISTALLISATIONSINHIBITOREN**
TRANSDERMAL THERAPEUTIC SYSTEM CONTAINING HORMONES AND CRYSTALLIZATION INHIBITORS
SYSTEME THERAPEUTIQUE TRANSDERMIQUE, CONTENANT DES HORMONES ET DES INHIBITEURS DE CRISTALLISATION

(30) Priorität: 25.06.1998 DE 19828273
(43) Veröffentlichungstag der Anmeldung: 11.04.2001
(73) Patentinhaber: LTS Lohmann Therapie-Systeme AG, 56626 Andernach (DE)
(72) Erfinder: KLEIN, Robert, Peter, D-56567 Neuwied (DE); MECONI, Reinhold, D-56567 Neuwied (DE); MÜLLER, Walter, D-56564 Neuwied (DE)
(74) Vertreter: Flaccus, Rolf-Dieter, Dr.
(86) Internationale Anmeldenummer: EP9903922
(87) Internationale Veröffentlichungsnummer: WO9966901

(56) Entgegenhaltungen:
- EP-A- 0 421 454
- EP-A- 0 531 938
- WO-A-95/09618
- WO-A-95/22322

## Beschreibung

Die Erfindung betrifft ein transdermales therapeutisches System (TTS) zur kontrollierten Abgabe von Estradiol in Kombination mit Norethisteronacetat an die menschliche Haut.

Estradiol in Kombination mit Norethisteronacetat zeigt in den zur Formulierung von transdermalen therapeutischen Systemen üblicherweise verwendeten Hilfsstoffen wie Polyacrylathaftklebern, Tackifiern, Weichmachern und Absorptionsverbesserern eine sehr geringe Sättigungslöslichkeit. Dadurch ist die Beladbarkeit von TTS mit gelöstem Wirkstoff stark begrenzt bzw. es treten während der Lagerung bei Übersättigung unerwünschte Kristallisationen auf. Dadurch wird der Anteil an gelösten Wirkstoffen in der Matrix reduziert, was sich negativ auf deren Freisetzung auswirkt.

Bei Kombinationspräparaten aus Estradiol und Norethisteronacetat wurden Anwendungsformen entwickelt, bei denen die Wirkstoffe in einem transdermalen therapeutischen System in räumlich getrennten Bereichen enthalten sind. Die Fertigung derartiger TTS ist jedoch sehr kostenaufwendig.

Das gemeinsame Einbringen von transdermalen therapeutischen Systemen mit Trocken-mitteln in die Primärpackung verringert die Gefahr der Rekristallisation, ist jedoch sehr aufwendig.

In DE-OS 43 36 557 ist ein wirkstoffhaltiges transdermales therapeutisches System auf Basis eines Haftklebers beschrieben, der Ester des Kolophoniums enthält. Die Herstellung erfolgt derart, daß die Komponenten bei Temperaturen zwischen 100 und 140 °C in der Schmelze geknetet und anschließend beschichtet werden. Diese hohen Temperaturen bei der Herstellung von Arzneiformen bergen die Gefahr, daß Abbauprodukte in einem unakzeptablen hohen Anteil gebildet werden können.

Die WO 95/30409 beschreibt ein topisches Polymer-Freisetzungssystem für die Verabreichung bestimmter Wirkstoffe mittels einer treibgasfreien Aerosolpumpe. Als Vorteil wird die Abwesenheit von Klebern herausgestellt. Als zusätzliche Komponenten werden Kristallisationsinhibitoren/Stabilisatoren und/oder Penetrationsenhancer wie substituierte Cyclodextrine, Transcutol, Harnstoff und Isoterpene verwendet, wobei die Wirkstoffkombination von Estradiol und Norethisteronacetat nicht beansprucht wird.

Aufgabe der Erfindung ist daher die Bereitstellung eines stabilen, d. h. rekristallisationsfreien Pflasters mit den Wirkstoffen Estradiol und Norethisteronacetat.

Überraschenderweise hat sich gezeigt, daß die Aufgabe durch ein transdermales therapeutisches System mit den Merkmalen des Hauptanspruches durch den Zusatz eines aminogruppenhaltigen Polymers als Kristallisationsinhibitor gelöst wird. Vorteilhaft werden als Kristallisationsinhibitoren Polymere auf Basis von Butylmethacrylat, 2-Dimethylaminoethylmethacrylat und Methylmethacrylat, vorzugsweise im molaren Verhältnis von 1 : 2 : 1, Polyaminoamide, Polyaminoimidazoline, Polyetherurethanamine, Polyamine und Polyglucosamine verwendet.
Es hat sich gezeigt, daß sich die Kristallisationsinhibitoren in einem Anteil von 0,05 bis 30 Gew.-% besonders eignen.

Durch Wasserstoffbrückenbildung zwischen den basischen Gruppen des Kristallisations-inhibitors und den beweglichen Wasserstoffatomen des Estradiol-Moleküls kommt es zu einer Immobilisierung von Estradiol. Dadurch wird die Konzentration an frei beweglichem Estradiol in der Matrix reduziert und die Kristallisation verhindert.

Das haftklebende Reservoir enthält Estradiol und Norethisteronacetat in einem Gewichtsverhältnis von 1 : 2 bis 1 : 15, vorzugsweise 1 : 3 bis 1 : 7, und einer Gesamtkonzentration von bis zu 25 Gew.-%.

Das Reservoir kann einen Bestandteil aus der Gruppe der Alterungsschutzmittel, Weichmacher, Antioxidantien und Absorptionsverbesserer, enthalten, wobei der Weichmacher in einer Konzentration von 0 bis 5 Gew.-% und das Alterungsschutzmittel in einer Konzentration von 0,1 bis 2 Gew.-% eingesetzt werden.

Geeignete Alterungsschutzmittel, Weichmacher, Antioxidantien und Absorptionsverbesserer sind dem Fachmann bekannt und beispielsweise in der DE 37 43 949 beschrieben.

Um das transdermale therapeutische System auf der Haut applizieren zu können, ist es erforderlich, daß dieses haftklebende Eigenschaften aufweist. Um diese Eigenschaften bei dem erfindungsgemäßen transdermalen therapeutischen System einzustellen, verwendet man Polyacrylathaftkleber in Form von Lösungen in organischen Lösungsmitteln, sogenannte Lösemittel-Haftkleber.

Weiterhin sind Polyacrylat-Haftkleber in Form von wäßrigen Dispersionen verwendbar.

Darüberhinaus sind auch Schmelzhaftkleber geeignet. Diese sind lösemittel- oder dispersionsmittelfrei und werden aus der Schmelze appliziert.

Weiterhin sind auch UV-vernetzbare Acrylathaftkleber geeignet. Diese sind lösemittelfrei und werden mit den üblichen Beschichtungsverfahren appliziert. Anschließend erfolgt durch Bestrahlung mit UV-Licht eine Vernetzung der Polymerketten. Dies ist erforderlich, um dem Haftkleber eine ausreichende Kohäsion zu geben.

Das Reservoir des transdermalen therapeutischen Systems kann aus mehreren Schichten bestehen, die gleiche oder unterschiedliche Wirkstoffkonzentrationen aufweisen können.

Die Schichtdicke des Reservoirs beträgt 0,02 mm bis 0,500 mm, vorzugsweise jedoch 0,030 mm bis 0,200 mm.

Das Reservoir kann mit einer zusätzlichen haftklebenden Schicht bzw. einem haftklebenden Rand versehen sein. Das wird dann erforderlich, wenn das Reservoir selbst nur unzureichende haftklebende Eigenschaften aufweist.

Das transdermale therapeutische System gemäß vorliegender Erfindung ist für therapeutische Zwecke in der Humanmedizin bestimmt.

Im folgenden wird die Erfindung anhand von Beispielen weiter erläutert.

### Beispiel 1

| | |
|---|---|
| 155,08 g | Durotak 387-2287 (Firma National Starch) (Polyacrylathaftkleber), |
| 4,81 g | Eudragit E 100 (Firma Röhm) (Polyacrlat) |

werden unter Rühren homogenisiert und mit der Aufschlämmung aus

| | |
|---|---|
| 2,17 g | Eutanol G (Firma Caesar und Lorentz) (langkettiger Fettalkohol) |
| 0,03 g | Aluminiumacetylacetonat (Firma Merck-Schuchardt), |
| 1,29 g | Estradiol-Hemihydrat, |
| 8,33 g | Norethisteronacetat |

in dem Lösemittelgemisch aus

| | |
|---|---|
| 27,98 g | Ethylacetat und |
| 27,97 g | Ethanol |

Die so erhaltene Kleberlösung wird auf eine wiederablösbare Schutzschicht Hostaphan RN 100, beidseitig silikonisiert, beschichtet, so daß nach dem Trocknen eine wirkstoffhaltige Matrix mit einem Flächengewicht von 96,3 g/m² resultiert. Auf die so erhaltene Matrix.wird eine für die Wirkstoffe undurchlässige Rückschicht (0,015 mm dicke Polyesterfolie) aufkaschiert. Anschließend werden 40 cm² große transdermale therapeutische Systeme ausgestanzt.

### Beispiele 2 - 7 und Vergleich:

Die Herstellung erfolgt, wie unter Beispiel 1 beschrieben, jedoch mit den in Tabelle 1 angegebenen Rohstoffen und Mengen.

**Tabelle 1:**

| Zusammensetzung [g] | | | | | | | |
|---|---|---|---|---|---|---|---|
| Beispiel | Vergleich | 2 | 3 | 4 | 5 | 6 | 7 |
| Durotak 387-2287 | 424,31 | 132,84 | 162,25 | 171,50 | 171,50 | 162,25 | 171,50 |
| Estradiol-Hemihydrat | 3,37 | 1,34 | 1,34 | 1,34 | 1,34 | 1,34 | 1,34 |
| Norethindronacetat | 21,60 | 8,65 | 8,65 | 8,65 | 8,65 | 8,65 | 8,65 |
| Eutanol G | 5,59 | 2,25 | 2,25 | 2,25 | 2,25 | 2,25 | 2,25 |
| Al-Acetylacetonat | 1,36 | 0,054 | 0,054 | 0,054 | 0,054 | 0,054 | 0,054 |
| Ethylacetat | | 36,48 | 29,28 | 27,11 | 27,11 | 29,28 | 27,11 |
| Ethanol | | 36,48 | 29,28 | 27,11 | 27,11 | 29,28 | 27,11 |
| Methylethylketon | 134,79 | -- | -- | -- | -- | -- | -- |
| Euredur 145 | -- | 20,0 | -- | -- | -- | -- | -- |
| Euredur 125 | -- | -- | 5,0 | -- | -- | -- | -- |
| Euredur 250 | -- | -- | -- | 0,5 | -- | -- | -- |
| Euredur 43 | -- | -- | -- | -- | 0,5 | -- | -- |
| Euredur 27 | -- | -- | -- | -- | -- | 5,0 | -- |
| Euredur 10 | -- | -- | -- | -- | -- | -- | 0,5 |

Die Prüfung auf Rekristallisationserscheinungen wurde mikroskopisch bei 40-facher Vergrößerung im Durchlicht durchgeführt. Die Ergebnisse sind in Tabelle 2 dargestellt.

**Tabelle 2:**

| Rekristallisationserscheinungen | |
|---|---|
| Beispiel 1 | Kristalle pro 40 cm² nach einer Lagerung von 3 Monaten bei 40 °C |
| Vergleich | 154 |
| 1 | 0 |
| 2 | 0 |
| 3 | 0 |
| 4 | 0 |
| 5 | 0 |
| 6 | 0 |
| 7 | 0 |

Wie die Tabelle 2 zeigt, erhält man durch den Zusatz von Kristallisationsinhibitoren transdermale therapeutische Systeme, die kristallisationsfrei sind, im Gegensatz zu dem Vergleichsbeispiel (ohne Kristallisationsinhibitor), bei dem es innerhalb eines Zeitraumes von 3 Monaten zu erheblicher Kristallisation kommt.

## Patentansprüche

1. Transdermales therapeutisches System in Pflasterform zur kontrollierten Abgabe von Estradiol in Kombination mit Norethisteronacetat, umfassend eine Rückschicht, ein damit verbundenes, Estradiol und Norethisteronacetat enthaltendes, an Wirkstoffen übersättigtes Reservoir, das unter Verwendung von Polyacrylat-Haftklebern und Kristallisationsinhibitoren hergestellt ist, und eine wiederablösbare Schutzschicht, **dadurch gekennzeichnet, daß** der Kristallisationsinhibitor ein aminogruppenhaltiges Polymer ist.

2. Transdermales therapeutisches System nach Anspruch 1, **dadurch gekennzeichnet, daß** der Kristallisationsinhibitor ausgewählt ist aus Polymeren auf Basis von Butylmethacrylat, 2-Dimethylaminoethylmethacrylat und Methylmethacrylat, insbesondere im molaren Verhältnis von 1 : 2 : 1, Polyaminoamiden, Polyaminoimidazolinen, Polyetherurethanaminen, Polyaminen und Polyglucosaminen.

3. Transdermales therapeutisches System nach einem der Ansprüche 1 oder 2, **dadurch gekennzeichnet, daß** das Reservoir einen oder mehrere Kristallisationsinhibitoren in einem Anteil von 0,05 - 30 Gew.% enthält.

4. Transdermales therapeutisches System nach einem oder mehreren der Ansprüche 1 - 3, **dadurch gekennzeichnet, daß** das Reservoir Estradiol und Norethisteronacetat in einem Gewichtsverhältnis von 1 : 2 bis 1 : 15, vorzugsweise 1 : 3 bis 1 : 7, und einer Gesamtkonzentration von bis zu 25 Gew.% enthält.

5. Transdermales therapeutisches System nach einem oder mehreren der Ansprüche 1 - 4, **dadurch gekennzeichnet, daß** das Reservoir einen Bestandteil aus der Gruppe der Alterungsschutzmittel, Weichmacher, Antioxydantien und Absorptionsverbesserer enthält, wobei der Weichmacher in einer Konzentration von 0 - 5 Gew.% und das Alterungsschutzmittel in einer Konzentration von 0,1 - 2 Gew.% eingesetzt werden.

6. Transdermales therapeutisches System nach einem oder mehreren der Anspruche 1 - 5, **dadurch gekennzeichnet, daß** der Haftkleber ein Lösemittelhaftkleber, Dispersionshaftkleber, Schmelzhaftkleber oder mit UV-Strahlen vernetzbarer Kleber ist.

7. Transdermales therapeutisches System nach einem oder mehreren der Ansprüche 1 - 6, **dadurch gekennzeichnet, daß** das Reservoir aus mehreren Schichten besteht.

8. Transdermales therapeutisches System nach einem oder mehreren der Ansprüche 1 - 7, **dadurch gekennzeichnet, daß** das Reservoir eine Schichtdicke von 0,02 mm - 0,500 mm, vorzugsweise von 0,030 - 0,200 mm aufweist.

9. Transdermales therapeutisches System nach einem oder mehreren der Ansprüche 1 - 8, **dadurch gekennzeichnet, daß** das Reservoir mit einer zusätzlichen haftklebenden Schicht bzw. einem haftklebenden Rand versehen ist.

## Claims

1. Transdermal therapeutic system in plaster form for controlled release of oestradiol in combination with norethisterone acetate, comprising a backing layer, a reservoir supersaturated with active ingredients and containing estradiol and norethisterone acetate, which reservoir is attached to said backing layer and is prepared using polyacrylate pressure-sensitive adhesives and crystallization inhibitors, and a detachable protective layer, **characterized in that** the crystallization inhibitor is an amino-containing polymer.

2. Transdermal therapeutic system according to Claim 1, **characterized in that** the crystallization inhibitor is selected from polymers based on butyl methacrylate, 2-dimethylaminoethyl methacrylate and methyl methacrylate, in particular in a molar ratio of 1:2:1, polyaminoamides, polyaminoimidazolines, polyetherurethaneamines, polyamines and polyglucosamines.

3. Transdermal therapeutic system according to either of Claims 1 and 2, **characterized in that** the reservoir comprises one or more crystallization inhibitors in a proportion of from 0.05-30% by weight.

4. Transdermal therapeutic system according to one or more of Claims 1 - 3, **characterized in that** the reservoir comprises oestradiol and norethisterone acetate in a weight ratio of from 1:2 to 1:15, preferably from 1:3 to 1:7, and in an overall concentration of up to 25% by weight.

5. Transdermal therapeutic system according to one or more of Claims 1 - 4, **characterized in that** the reservoir includes a constituent from the group of ageing inhibitors, plasticizers, antioxidants and absorption improvers, the plasticizer being used in a concentration of 0-5% by weight and the ageing inhibitor in a concentration of 0.1-2% by weight.

6. Transdermal therapeutic system according to one or more of Claims 1 - 5, **characterized in that** the pressure-sensitive adhesive is a solvent-based adhesive, a dispersion adhesive, a hot-melt adhesive or a UV-crosslinkable adhesive.

7. Transdermal therapeutic system according to one or more of Claims 1 - 6, **characterized in that** the reservoir consists of two or more layers.

8. Transdermal therapeutic system according to one or more of Claims 1 - 7, **characterized in that** the reservoir has a layer thickness of 0.02 mm-0.500 mm, preferably 0.030-0.200 mm.

9. Transdermal therapeutic system according to one or more of Claims 1 - 8, **characterized in that** the reservoir is provided with an additional pressure-sensitive adhesive layer and/or with a pressure-sensitive adhesive margin.

## Revendications

1. Système thérapeutique transdermique en forme d'emplâtre pour la libération contrôlée d'oestradiol en combinaison avec de l'acétate de noréthistérone, comprenant une couche dorsale, un réservoir sursaturé en substances actives contenant de l'oestradiol et de l'acétate de noréthistérone reliés à la première citée, qui a été préparé en utilisant des agents auto-adhésifs à base de polyacrylate et des inhibiteurs de la cristallisation et une couche de protection amovible, **caractérisé en ce que** l'inhibiteur de la cristallisation est un polymère contenant des groupes amino.

2. Système thérapeutique transdermique selon la revendication 1, **caractérisé en ce que** l'inhibiteur de la cristallisation est choisi parmi des polymères à base de méthacrylate de butyle, de méthacrylate de 2-diméthylaminoéthyle et de méthacrylate de méthyle en particulier dans le rapport molaire de 1 : 2 : 1, des polyaminoamides, des polyaminoimidazolines, des polyétheruréthanamines, des polyamines et des polyglucosamines.

3. Système thérapeutique transdermique selon la revendication 1 ou 2, **caractérisé en ce que** le réservoir contient un ou plusieurs inhibiteurs de la cristallisation en une fraction de 0,05 - 30 % en poids.

4. Système thérapeutique transdermique selon une ou plusieurs des revendications 1 à 3, **caractérisé en ce que** le réservoir contient de l'oestradiol et de l'acétate de noréthistérone dans un rapport pondéral de 1 : 2 à 1 : 15, de préférence de 1 : 3 à 1 : 7 et en une concentration totale s'élevant jusqu'à 25 % en poids.

5. Système thérapeutique transdermique selon une ou plusieurs des revendications 1 à 4, **caractérisé en ce que** le réservoir contient un constituant choisi parmi le groupe comprenant des agents de protection contre le vieillissement, des plastifiants, des antioxydants et des agents améliorant l'absorption, le plastifiant étant mis en oeuvre en une concentration de 0 à 5 % en poids et l'agent de protection contre le vieillissement étant mis en oeuvre en une concentration de 0,1 à 2 % en poids.

6. Système thérapeutique transdermique selon une ou plusieurs des revendications 1 à 5, **caractérisé en ce que** l'agent auto-adhésif est un agent auto-adhésif à application par solvant, un agent auto-adhésif à dispersion, un agent auto-adhésif thermofusible ou une colle réticulable par exposition à un rayonnement ultraviolet.

7. Système thérapeutique transdermique selon une ou plusieurs des revendications 1 à 6, **caractérisé en ce que** le réservoir est constitué par plusieurs couches.

8. Système thérapeutique transdermique selon une ou plusieurs des revendications 1 à 7, **caractérisé en ce que** le réservoir présente une épaisseur de couche de 0,02 mm - 0,500 mm, de préférence de 0,030 mm - 0,200 mm.

9. Système thérapeutique transdermique selon une ou plusieurs des revendications 1 à 8, **caractérisé en ce que** le réservoir est muni d'une couche auto-adhésive supplémentaire, respectivement d'un bord auto-adhésif.
